# EUROPEAN PATENT SPECIFICATION

(11) **EP 2 411 355 B1**
(45) Date of publication and mention of the grant of the patent: **16.01.2013**
(21) Application number: 10713719.2
(22) Date of filing: 19.03.2010
(51) Int. Cl.: C07C 33/12, C07C 69/608, C11B 9/00

(54) **Alcohol as sandalwood odorant**
Alkohol als Sandelholz-Duftstoff
Alcool en tant que matière odorante à note de bois de santal

(30) Priority: 24.03.2009 WO PCT/IB2009/051223
(43) Date of publication of application: 01.02.2012
(73) Proprietor: Firmenich S.A., 1211 Geneva 8 (CH)
(72) Inventor: PAMINGLE, Hervé, CH-1290 Versoix (CH); CHAPUIS, Christian, CH-1295 Mies (CH); FANKHAUSER, Peter, CH-1217 Meyrin (CH); FANTINI, Piero, CH-1255 Veyrier (CH)
(74) Representative: Carina, Riccardo Filippo
(86) International application number: PCT/IB2010/051199
(87) International publication number: WO 2010/109391

(56) References cited:
- US-A- 5 696 075
- US-B1- 6 235 943

## Description

### Technical field

The present invention relates to the field of perfumery. More particularly, it concerns some derivatives of the 2-ethyl-4-(2',2'-dimethyl-3'-alkyl-3'-cyclopenten-1'-yl)-4-penten-1-ol type, as defined herein below. Said compounds are very interesting perfuming ingredients due to their unusual combination of sandalwood and floral notes.

The present invention concerns the use of said compounds in the perfumery industry as well as the compositions or articles containing said compounds.

### Prior art

To the best of our knowledge, none of the invention's compounds has been reported in the prior art.

Amongst the known perfuming ingredients, we refer to the structural analogues cited in the document US 4610813 or US 5504066.

Document US 4610813 discloses a limited number of compounds wherein the carbon atom number 2 of the 4-penten-1-ol moiety is substituted by a methyl group, two methyl groups or a methyl and an ethyl group, and in particular 2-methyl-4-(2',2'-dimethyl-3'-methyl-3'-cyclopenten-1'-yl)-4-penten-1-ol. Document US 6235943 discloses perfuming ingredients with a similar compounds backbone but different positioning of the substituents. However, all the compounds disclosed in said documents possess odor significantly different from that of the present compounds. Therefore said documents do not report or suggest any organoleptic properties of the present compounds of formula (I), or any use of said compounds in the field of perfumery.

Document US 5504066 discloses compounds wherein the carbon atom number 2 of the 4-penten-1-ol moiety is substituted by two methyl or ethyl groups, and in particular discloses 2,2-dimethyl-4-(2',2'-dimethyl-3'-methyl-3'-cyclopenten-1'-yl)-4-penten-1-ol. Said document also discloses a very general formula embracing some of the present compounds, but does not specifically disclose our present 2-mono-substituted derivatives, nor our preferred embodiments, which are all limited to 2-di-substituted compounds. Furthermore, nothing in US 5504066 suggests that said compounds could even have a floral note. Therefore said document does not report or suggest the odor properties of the present compounds of formula (I), or even that said present compounds are of potential real and significant interest for perfumery, which is clearly different from just having an odor.

### Description of the invention

We have now surprisingly discovered that some derivatives of the 2-ethyl-4-(2',2'-dimethyl-3'-alkyl-3'-cyclopenten-1'-yl)-4-penten-1-ol type, i.e. a compound of formula 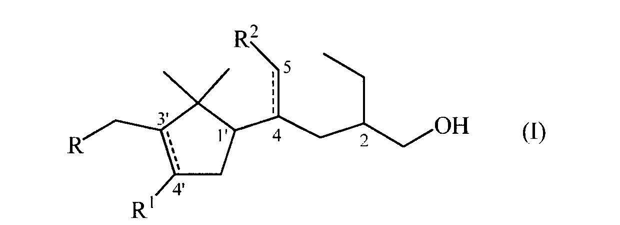
wherein R is a hydrogen atom or a methyl group;
R² represents a hydrogen atom and the bond between the carbon atoms 4 and 5 is a double bond, or R² represents a CH₂ group forming with the carbon atoms 4 and 5 a cyclopropane ring; and
R¹ represents a hydrogen atom and the bond between the carbon atoms 3' and 4' is a single or double bond, or R¹ represents a CH₂ group forming with the carbon atoms 3'
and 4' a cyclopropane ring;
can be used as perfuming ingredient, for instance to impart odor notes of the floral and sandalwood type. This type of association (floral and sandalwood) is at least very unusual in perfumery, in fact to the best of our knowledge is it reported here for the first time.

It is understood that the present invention concerns the compounds of formula (I) in the form of any one of its enantiomers or in the form of a mixture of said enantiomers (compound optically enriched or racemate).

According to a particular embodiment of the invention, the compounds of formula (I) are those of formula 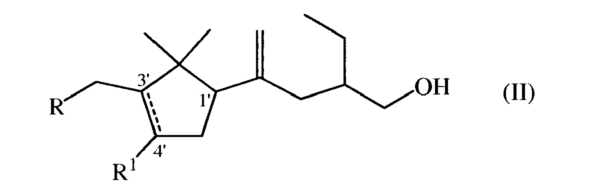
wherein R is a hydrogen atom or a methyl group; and
R¹ represents a hydrogen atom and the bond between the carbon atoms 3' and 4' is a single or double bond, or R¹ represents a CH₂ group forming with the carbon atoms 3' and 4' a cyclopropane ring.

According to a further embodiment of the invention, said compound (II) is a compound wherein R¹ is a hydrogen atom. In particular a compound wherein R¹ is a hydrogen atom and the bond between the carbon atoms 3' and 4' is a double bond.

According to a particular embodiment of the present invention, said compound (I) or (II) is a compound having the carbon atom 1' with a stereo configuration of the type (R) (optically actif). or at least having said carbon atom 1' with a stereo configuration ® and an e.e. (enantiomeric excess) of above 50 %, preferably above 70%.

Amongst the invention's compounds, one may cite (1'R)-2-ethyl-4-(2',2',3'-trimethyl-3'-cyclopenten-1'-yl)-4-penten-1-ol, which is one of the most appreciated by the perfumer. This compound possesses a very diffusive and rising sandalwood-woody note which is associated with a nice floral-rosy note, this latter note influencing significantly the perfumistic performance of the invention's compound.

Other compounds of formula (I) are also described in Table (I) herein below, together with their odours:

**Table 1 : Structure and odour characteristics of the invention's compounds**

| Structure of compound (I) | Odor |
|---|---|
| 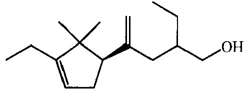 | A well balanced and natural sandalwood note having also a floral, slightly herbaceous, note of the jasmine-lilly type. |
| (1'R)-2-ethyl-4-(3'-ethyl-2'.2'-dimethyl-3'-cyclopenten-1'-yl)-4-penten-1-ol | |
| 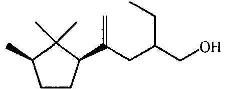 | Sandalwood, cedar, with a floral note of the phenylacetaldehyde glycerylacetal type (Arctander N° 2488) |
| (1'*R*,3'*R*)-2-ethyl-4-(2',2',3'-trimethyl-1'-cyclopentyl)-4-penten-1-ol | |
| 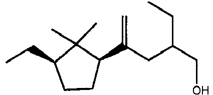 | Woody-sandalwood, floral |
| (-)-2-ethyl-4-((1*R'*,3*R'*)-3-ethyl-2,2-dimethylcyclopentyl)pent-4-en-1-ol | |
| 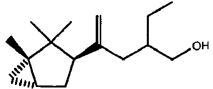 | Woody-sandalwood, floral, with an aspect of the ambery type |
| (-)-2-ethyl-4-((1S,3R,SR)-1,2,2-trimethylbicyclo[3.1.0]hexan-3-yl)pent-4-en-1-ol | |

The invention's compounds, due to their unusual combination of odor notes, are particularly useful for those perfumery applications where both sandalwood and floral notes are important.

When the odor of the invention's compounds is compared with the one of the prior art compounds, such as the one disclosed in US 4610813 or US 5504066, then the invention's compounds distinguish themselves by possessing a significant and characteristic floral type notes. Said differences lend the invention's compounds and the prior art compounds to be each suitable for different uses, i.e. to impart different organoleptic impressions.

In particular the odor of (1'R)-2-ethyl-4-(2',2',3'-trimethyl-3'-cyclopenten-1'-yl)-4-penten-1-ol distinguishes itself from those of 2-methyl-4-(2'.2'-dimethyl-3'-methyl-3'-cyclopenten-1'-yl)-4-penten-1-ol (the closest analogue) by having a rosy note which is totally absent in the prior art compound, as well as by having a different sandalwood note, i.e. that of the invention's compound is much more light-woody, substantive and diffusive, while that of the prior art compound is much more heavy, fatty and sandal milk. The examples will further show these differences.

As mentioned above, the invention concerns the use of a compound of formula (I) as perfuming ingredient. In other words it concerns a method to confer, enhance, improve or modify the odor properties of a perfuming composition or of a perfumed article, which method comprises adding to said composition or article an effective amount of at least a compound of formula (I). In particular said use is intended to impart sandalwood and floral notes. By "use of a compound of formula (I)" it has to be understood here also the use of any composition containing compound (I) and which can be advantageously employed in perfumery industry as active ingredients.

Said compositions, which in fact can be advantageously employed as perfuming ingredients, are also an object of the present invention.

Therefore, another object of the present invention is a perfuming composition comprising:
i) as perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" we mean here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting example solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery base, other suitable perfumery carriers, than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company).

As solid carrier one may cite, as non-limiting examples, absorbing gums or polymers, or yet encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloids : Stabilisatoren, Dickungs- und Gehermittel in Lebensmittel, Band 2 der Schriftenreihe Lebensmittelchemie, Lebensmittelqualität, Behr's VerlagGmbH & Co., Hamburg, 1996. The encapsulation is a well known process to a person skilled in the art, and may be performed, for instance, using techniques such as spray-drying, agglomeration or yet extrusion ; or consists of a coating encapsulation, including coacervation and complex coacervation techniques.

By "perfumery base" we mean here a composition comprising at least one perfuming co-ingredient.

Said perfuming co-ingredient is not of the formula (I). Moreover, by "perfuming co-ingredient" it is meant here a compound, which is used in perfuming preparation or composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of its general knowledge and according to intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

An invention's composition consisting of at least one compound of formula (I) and at least one perfumery carrier represents a particular embodiment of the invention as well as a perfuming composition comprising at least one compound of formula (I), at least one perfumery carrier, at least one perfumery base, and optionally at least one perfumery adjuvant.

It is useful to mention here that the possibility to have, in the compositions mentioned above, more than one compound of formula (I) is important as it enables the perfumer to prepare accords, perfumes, possessing the odor tonality of various compounds of the invention, creating thus new tools for his work.

For the sake of clarity, it is also understood that any mixture resulting directly from a chemical synthesis, e.g. a reaction medium without an adequate purification, in which the compound of the invention would be involved as a starting, intermediate or end-product could not be considered as a perfuming composition according to the invention as far as said mixture does not provide the inventive compound in a suitable form for perfumery.

Furthermore, the invention's compound can also be advantageously used in all the fields of modern perfumery, i.e. fine or functional perfumery, to positively impart or modify the odor of a consumer product into which said compound (I) is added. Consequently, a perfuming consumer product which comprises :
i) as perfuming ingredient, at least one compound of formula (I), as defined above; and
ii) a fine or functional perfumery base ;
is also an object of the present invention.

For the sake of clarity, it has to be mentioned that, by "fine or functional perfumery base" we mean here a consumer product which is compatible with perfuming ingredients and is expected to deliver a pleasant odor to the surface to which it is applied (e.g. skin, hair, textile, or home surface). In other words, a consumer product for the purpose of perfuming according to the invention comprises the functional formulation, as well as optionally additional benefit agents, corresponding to the desired consumer product, e.g. a detergent or an air freshener, and an olfactive effective amount of at least one invention's compound.

The nature and type of the constituents of the fine or functional perfumery base do not warrant a more detailed description here, which in any case would not be exhaustive, the skilled person being able to select them on the basis of his general knowledge and according to the nature and the desired effect of said product.

Non-limiting examples of suitable fine or functional perfumery base can be a perfume, such as a fine perfume, a cologne or an after-shave lotion; a fabric care product, such as a liquid or solid detergent, a fabric softener, a fabric refresher, an ironing water, a paper, or a bleach; a body-care product, such as a hair care product (e.g. a shampoo, a coloring preparation or a hair spray), a cosmetic preparation (e.g. a vanishing cream or a deodorant or antiperspirant), or a skin-care product (e.g. a perfumed soap, shower or bath mousse, oils or gel, or a hygiene product); an air care product, such as an air freshener or a "ready to use" powdered air freshener; or a home care product, such as a wipe, a dish detergent or hard-surface detergent.

Some of the above-mentioned consumer product bases may represent an aggressive medium for the invention's compound, so that it may be necessary to protect the latter from premature decomposition, for example by encapsulation or by chemically bounding it to another chemical which is suitable for releasing the invention's ingredient upon a suitable external stimulus, such as an enzyme, light, heat or a change of pH.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0. 1 % to 15 % by weight, or even more, of the compounds of the invention based on the weight of the composition into which they are incorporated. Concentrations lower than these, such as in the order of 0.01% to 10% by weight, can be used when these compounds are incorporated into perfumed articles, percentage being relative to the weight of the article.

The invention's compounds can be prepared according to a method as described in the examples hereinbelow, and using the campholenic aldehyde or a derivative thereof as staring material. The synthetic schema can be resumed as follows :

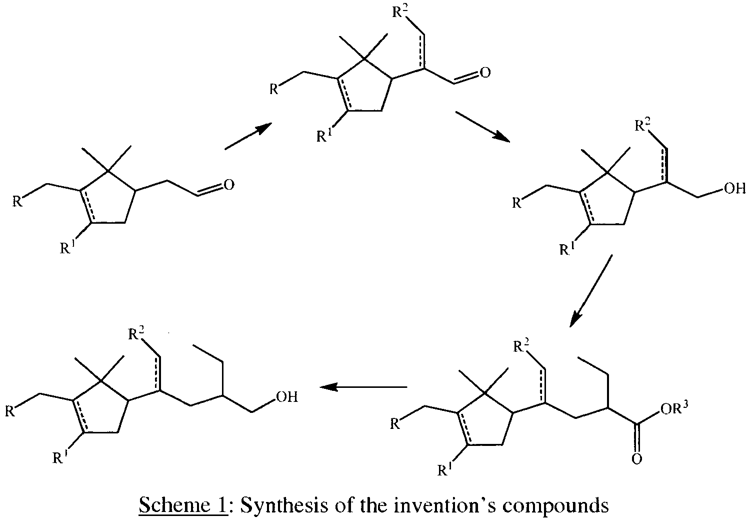

The key intermediate is the ester of formula 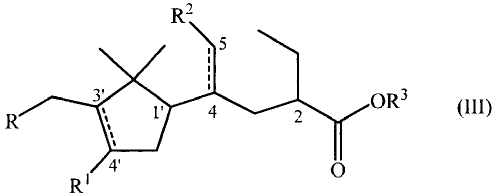 wherein R is a hydrogen atom or a methyl group;
R² represents a hydrogen atom and the bond between the carbon atoms 4 and 5 is a double bond, or R² represents a CH₂ group forming with the carbon atoms 4 and 5 a cyclopropane ring;
R¹ represents a hydrogen atom and the bond between the carbon atoms 3' and 4' is a single or double bond, or R¹ represents a CH₂ group forming with the carbon atoms 3' and 4' a cyclopropane ring; and
R³ represents a C₁₋₄ alkyl group;
in the form of any one of its enantiomers or in the form of a mixture of said enantiomers;
which is also a new compound, and therefore another object of the present invention, as valuable intermediate in the preparation of the invention's compound.

According to a particular embodiment of said ester are of formula 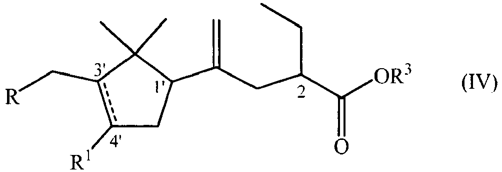 wherein R is a hydrogen atom or a methyl group; and
R¹ represents a hydrogen atom and the bond between the carbon atoms 3' and 4' is a single or double bond, or R² represents a CH₂ group forming with the carbon atoms 3' and 4' a cyclopropane ring; and
R³ represents a C₁₋₄ alkyl group.

According to a further embodiment of the invention, said compound (III) or (IV) is a compound wherein R¹ is a hydrogen atom. In particular a compound wherein R¹ is a hydrogen atom and the bond between the carbons atom 3' and 4' is a double bond.

According to a particular embodiment of the present invention said compound (III) or (IV) is a compound having the carbon atom 1' with a stereo configuration of the type (R) (optically actif), or at least having said carbon atom 1' with a stereo configuration ® and an e.e. (enantiomeric excess) of above 50 %, preferably above 70%.

### Examples

The invention will now be described in further detail by way of the following examples, wherein the abbreviations have the usual meaning in the art, the temperatures are indicated in degrees centigrade (°C) ; the NMR spectral data were recorded in CDCl₃ (if not stated otherwise) with a 360 or 400 MHz machine for ¹H and ¹³C, the chemical shifts δ are indicated in ppm with respect to TMS as standard, the coupling constants J are expressed in Hz. α_{D}²⁰ were measured for the pure compound, unless otherwise specified (e.g. in solution).

### Example 1

### Synthesis of compounds of formula (I)

1) (-)-(1'*R*)-2-ethyl-4-(3'-ethyl-2',2'-dimethyl-3'-cyclopenten-1'-yl)-4-penten-1-ol
   *a) (-)-(S)-2-(3-ethyl-2,2-dimethyl-3-cylopenten-1-yl)-2-propenal* Bu₂NH (1.41g, 11 mmol) was added to a mixture of aldehyde (+)-3-ethyl-2,2-dimethyl-3-cyclopentene-1-acetaldehyde [described in C. Chapuis, R. Brauchli, *Helv. Chim. Acta* **1992**, *75*, 1527] (30g, 180 mmol) and 36% H₂CO/H₂O (12.17g, 256 mmol) at reflux. After 3 hours the cold reaction mixture was extracted with Et₂O. The organic phase was washed to neutrality with H₂O, dried (Na₂SO₄), concentrated and distilled through a *Vigreux* column to afford pure the desired product.
      Bp: 78°C/4.8 mbar. α_{D}²⁰ = -118.2.
      ¹H-NMR: 0.69 (*s*, 3H); 1.04 (*s,* 3H); 1.09 (*t*, *J*=7, 3H); 1.92 (*m*, 2H); 2.37 (*m,* 2H); 3.22 (*t, J*=8, 1H); 5.31 (*m,* 1H); 6.12 (*s*, 1H); 6.35 (s, 1H); 9.58 (*s*, 1H).
      ¹³C-NMR: 12.2 (*q*); 19.8 (t); 22.0 (*q*); 26.7 (*q*); 34.7 (*t*); 46.8 (*d*); 48.4 (*s*); 119.0 (*d*); 135.6 (*t*); 151.3 (*s*); 153.4 (*s*); 195.3 (*d*).
   *b) (-)-(S)-2-(3-ethyl-2,2-dimethyl-3-cyclopenten-1-yl)-2-propen-1-ol*
      A solution of (-)-(*S*)-2-(3-ethyl-2,2-dimethyl-3-cyclopenten-1-yl)-2-propenal (21g, 118 mmol) in Et₂O (24 ml) was added dropwise to a suspension of LiAlH₄ (2.24g, 59 mmol) in Et₂O (36 ml). After 45 minutes, H₂O (2.24 ml), 15% NaOH (2.24 ml), H₂O (6.72 ml) were added successively. The mixture was filtered, dried (Na₂SO₄), concentrated, distilled through a *Vigreux* column to afford pure desired product in 58 % yield.
      Bp: 60°C/4 mbar. (α_{D}²⁰ = -80.3.
      ¹H-NMR: 0.78 (*s*, 3H); 1.08 (*s*, 3H); 1.09 (*t*, *J* = 7, 3H); 1.92 (*m*, 2H); 1.98 (br,*s*, OH); 2.32 (*m*, 2H); 2.58 (*t*, *J*=8, 1H); 4.10 (br*q*, *J*=12, 2H); 4.99 (br,s, 1H); 5.22 (*q, J*=1, 1H); 5.29 (*quint*, *J*=1, 1H).
      ¹³C-NMR: 12.2 (*q*); 19.8 (*t*); 21.4 (*q*); 26.9 (*q*); 33.9 (*t*); 48.0 (*s*); 54.3 (*d*); 66.2 (*t*); 110.6 (*t*); 119.2 (*d*); 149.4 (*s*); 153.5 (*s*).
   *c) (-)-methyl (1'R)-2-ethyl-4-(3'-ethyl-2',2'-dimethyl-3'-cyclopenten-1'-yl)-4-pentenoate*
      A mixture of alcohol obtained in b) (2.5g, 14 mmol), pivalic acid (0.14g, 1.4 mmol) and trimethylorthobutyrate (6.17g, 42 mmol) was heated at 90°C for 1 h. then slowly to 160°C in 4.5 h. with continuous distillation of EtOH. The cold reaction mixture was washed to neutrality with H₂O, dried (Na₂SO₄), concentrated and bulb-to-bulb distilled to afford pure desired product in 60% yield as a 41:59 mixture.
      Bp^{:} 90°C/0.28 mbar. α_{D}²⁰ = -68.2.
      ¹H-NMR: major 0.73 (s, 3H); 0.91 (*t, J* = 7, 3H); 1.04 (*s*, 3H); 1.05 (*t*, *J*=7, 3H); 1.54 (*m,* 1H); 1.62 (*m*, 1H); 1.92 (*m,* 2H); 2.2 (*m,* 1H); 2.3 (*m*, 1H); 2.4 (*m*, 1H); 2.55 (*m*, 1H); 3.63 (*s,* 3H); 4.9 (*s*, 2H); 5.28 (*m*, 1H); minor 0.75 (*s*, 3H); 0.89 (*t*, *J*=7, 3H); 1.04 (*s*, 3H); 1.05 (*t*, *J*=7, 3H); 1.54 (*m*, 1H); 1.62 (*m*, 1H); 1.92 (*m*, 2H); 2.2 (*m*, 1H); 2.3 (*m*, 1H); 2.4 (*m*, 1H); 2.55 (*m*, 1H); 3.67 (*s*, 3H); 4.87 (*s*, 1H); 4.9 (*s*, 1H); 5.28 (*m*, 1H).
      ¹³C-NMR: major 12.0 (*q*); 12.2 (*q*); 19.8 (*t*); 21.4 (*q*); 25.9 (*t*); 27.2 (*q*); 34.3 (*t*); 39.1 (*t*); 46.3 (*d*); 48.2 (*s*); 51.2 (*q*); 56.1 (*d*); 111.8 (*t*); 119.2 (*d*); 147.2 (*s*); 153.4 (*s*); 176.3 (*s*);
      minor 11.8 (*q*); 12.2 (*q*); 19.8 (*t*); 21.3 (*q*); 25.4 (*t*); 26.9 (*q*); 34.1 (*t*); 40.0 (*t*); 46.0 (*d*); 48.0 (*s*); 51.3 (*q*); 56.5 (*d*); 112.4 (*t*); 119.0 (*d*); 147.1 (*s*); 153.5 (*s*); 176.5 (*s*).
   *d) (-)-(1'R)-2-ethyl-4-(3'-ethyl-2',2'-dimethyl-3'-cyclopenten-1'-yl)-4-penten-1-ol*
      A solution of the ester obtained under c) (2.1g, 8 mmol) in Et₂O (1.6 ml) was added to a suspension of LiAlH₄ (0.23g, 6 mmol) in Et₂O (2.4 ml). After 1 hour, H₂O (0.23 ml), 15% NaOH (0.23 ml), H₂O (0.69 ml) were added successively. The mixture was filtered, dried (Na₂SO₄), concentrated, and bulb-to-bulb distilled to afford pure desired product in 88% yield as a 41:59 mixture.
      Bp: 105°C/0.13 mbar. α_{D}²⁰ = -80.6.
      ¹H-NMR: major 0.65 (*s*, 3H); 0.93 (*t*, *J*=7, 3H); 1.04 (*s*, 3H); 1.08 (*t, J*=7, 3H); 1.32 (*m*, 2H); 1.7 (*m*, 1H); 1.92 (*m*, 2H); 2.05 (*m*, 1H); 2.17 (*m*, 1H); 2.20 (*m*, 1H); 2.35 (*m*, 1H); 2.58 (*m*, 1H); 3.58 (*m*, 2H); 4.92 (*s*, 2H); 5.29 (*m*, 1H);
      minor 0.66 (*s*, 3H); 0.91 (*t, J*=7_{.} 3H); 1.02 (*t*, *J*=7, 3H); 1.14 (*s*, 3H); 1.42 *(m,* 2H); 1.7 (*m*, 1H); 1.92 (*m,* 2H); 2.05 (*m,* 1H); 2.17 (*m*, 1H); 2.20 (*m,* 1H); 2.35 *(m,* 1H); 2.58 *(m,* 1H); 3.58 *(m,* 2H); 4.92 (s, 1H); 4.98 (s, 1H); 5.29 *(m,* 1H).
      ¹³C-NMR: major 11.2 (*q*); 12.2 (*q*); 19.8 (*t*); 21.5 (*q*); 24.4 (*t*); 27.2 (*q*); 34.4 (*t*); 39.3 (*t*); 40.6 (*d*); 48.1 (*s*); 55.3 (*d*); 65.3 (*t*); 112.1 (*t*); 119.1 (*d*); 149.1 (*s*); 153.5 (*s*);
      minor 11.5 (*q*); 12.2 (*q*); 19.8 (*t*); 21.5 (*q*); 23.2 (*t*); 27.0 (*q*); 34.3 (*t*); 40.3 (*t*); 40.3 (*d*); 48.1 (*s*); 56.1 (*d*); 65.3 (*t*); 112.3 (*t*); 119.1 (*d*); 148.1 (*s*); 153.5 (*s*).
2) (-)-(1'*R*)-2-ethyl-4-(2',2',3'-trimethyl-3'-cyclopenten-1'-yl)-4-penten-1-ol
   *a) (-)-methyl (1'R)-2-ethyl-4-(2',2',3'-trimethyl-3'-cyclopenten-1'-yl)-4-pentenoate* Obtained in 80% yield as a 40:60 mixture of diastreomeres from (-)-2-(2,2,3-trimethyl-3-cyclopenten-1-yl)-2-propen-1-ol [described in US5696075] according to the procedure used under 1-c).
      Bp: 85°C/0.3 mbar. α_{D}²⁰ = -46.3.
      ¹H-NMR: major 0.72 (*s*, 3H); 0.90 (*t, J* = 7, 3H), 1.10 (*s*, 3H); 1.52-1.65 (*m*, 1H); 1.60 (*s*, 3H); 2.18 (m, 2H); 2.30 (m, 1H); 2.42 (m, 1H); 2.55 (m, 2H); 3.63 (*s*, 3H); 4.84 (*s*, 1H); 4.89 (*s*, 1H); 5.26 (s, 1H);
      minor 0.74 (*s*, 3H); 0.88 (*t, J* = 7, 3H); 1.08 (*s*, 3H); 1.52-1.65 (*m,* 2H); 1.60 (*s*, 3H); 2.18 (m*,* 2H); 2.30 (m, 1H); 2.42 (m, 1H); 2.55 (m, 2H); 3.68 (*s*, 3H); 4.89 (*s*, 1H); 4.90 (s, 1H); 5.27 (s, 1H).
      ¹³C-NMR: major 176.3 (*s*); 147.2 (*s*); 147.2 (*s*); 121.6 (*d*); 111.7 (*t*); 55.6 (*d*); 51.2 (*q*); 47.7 (*s*); 46.0 (*d*); 39.9 (*t*); 34.2 (*t*); 27.0 (*q*); 25.9 (*t*); 21.0 (*q*), 12.8 (*q*); 12.0 (*q*);
      minor 176.5 (*s*); 147.4 *(s);* 147.2 (*s*); 121.5 (*d*); 112.2 (*t*); 56.0 (*d*); 51.3 (*q*); 47.9 (*s*); 46.2 (*d*); 39.0 (*t*); 34.0 (*t*); 26.8 (*q*); 25.3 (*t*); 20.9 (*q*); 12.8 (*q*); 11.8 (*q*).
   b) *(-)-(1'R)-2-ethyl-4-(2',2',3'-trimethyl-3'-cyclopenten-1'-yl)-4-penten-1-ol* Obtained in 87% yield as a 40:60 mixture of diastreomeres, according to the procedure used under 1-d).
      Bp: 59°C/0_{.}1 mbar. α_{D}²⁰ = -50.7.
      ¹H-NMR: Major 5.27 (*s*, 1H); 4.93 (*s*, 2H); 3.56 (*m,* 2H); 2.57 (*m,* 1H); 2.35 (*m,* 1H); 2.17 (*m,* 2H); 2.05 (*m,* 1H); 1.66 (*m,* 1H); 1.63 (brs, OH); 1.60 (*s*, 3H); 1.44 (*m*, 1H); 1.32 (*m*, 1H); 1.11 (*s*, 3H); 0.94 (*t*, *J*=7, 3H); 0.76 (s, 3H)
      minor 5.27 (*s*, 1H); 4.97 (*s*, 1H); 4.93 (*s*, 1H); 3.56 (*m,* 2H); 2.57 (*m,* 1H); 2.35 (*m*, 1H); 2.17 (*m,* 2H); 2.05 (*m,* 1H); 1.66 (*m,* 1H); 1.63 (br,s, OH); 1.60 (*s*, 3H); 1.44 (*m,* 1H); 1.32 (*m,* 1H); 1.10 (*s*, 3H); 0.90 (*t, J* = 7, 3H); 0.76 (*s*, 3H).
      ¹³C-NMR: major 149.1 (*s*); 148.4 (*s*); 122.0 (*d*); 112.2 (*t*); 65.4 (*t*); 55.6 (*d*); 47.7 (*s*); 40.6 (*d*); 40.1 (*t*); 34.1 (*t*); 26.9 (*q*); 23.2 (*t*); 21.0 (*q*); 12.8 (*q*); 11.5 (*q*); minor 148.4 (*s*); 147.4 (*s*), 122.0 (*d*); 112.2 (*t*); 65.4 (*t*); 54.9 (*d*); 47.7 (*s*); 40.3 (*d*); 39.2 (*t*); 34.3 (*t*); 27.1 (*q*); 24.4 (*t*); 21.0 (*q*); 12.8 (*q*); 11.1 (*q*)*.*
3) (+)-(1'*R*,3'*R*)-2-ethyl-4-(2',2'.3'-trimethyl-1'-cyclopentyl)-4-penten-1-ol
   *a) methyl (1'R,3'R)-2-ethyl-4-(2,2,3-trimethyl-1'-cyclopentyl)-4-pentenoate* A solution of ester obtained under 2-a) (1.1g, 4.4 mmol), in EtOH (11 ml) was hydrogenated (110 ml H₂) over *Raney-Ni* (0.1g) during 48 hours. The reaction mixture was filtered, concentrated, and bulb-to-bulb distilled to afford pure desired product in 95% yield.
      Bp: 150°C/0.1 mbar.
      MS: 252 (5, M^{+.}), 237 (12), 209 (12), 205 (10), 177 (21), 169 (18), 149 (21), 137 (32), 109 (100), 96 (61), 81 (32), 68 (48), 55 (36), 41 (44).
   *b) (+)-(1'R,3'R)-2-ethyl-4-(2,2',3'-trimethyl-1'-cyclopentyl)-4-penten-1-ol* Methyl ester obtained under a) (0.91g, 3.6 mmol) was reduced with LiAlH₄ (137 mg, 3.6 mmol) in Et₂O (10 ml) according to the procedure used under 1-d), to afford a 1.1:1 mixture of stereoisomers of the desired product in 89% yield. Bp: 100°C/0.05 mbar. α_{D}²⁰ = +2.5.
      ¹H-NMR: major 4.99 (*s*, 1H); 4.83 (*s*, 1H); 3.56 (*m,* 2H); 2.17 (*m*, 2H); 2.04 (m, 2H); 1.77 (*m*, 1H); 1.67 (*m,* 2H); 1.60 (m, 1H); 1.49 (brs, OH); 1.40 *(m,* 1H); 1.30 *(m,* 1H); 1.24 (*m*, 1H); 0.93 *(q,* J = 7, 3H); 0.92 (s, 3H); 0.85 (*d*, *J* = 7, 3H); 0.55 (s, 3H); minor 4.94 (*s*, 1H); 4.85 (*s*, 1H); 3.56 *(m,* 2H); 2.17 *(m,* 2H); 2.04 *(m,* 2H); 1.77 (*m*, 1H); 1.67 *(m,* 2H); 1.60 *(m,* 1H); 1.49 (brs, OH); 1.40 *(m,* 1H); 1.30 (*m,* 1H); 1.24 (*m*, 1H); 0.94 (*s*, 3H); 0.92 (*q, J* = 7,3H); 0.84 (*d, J* = 7, 3H); 0.55 (*s*, 3H).
      ¹³C-NMR: major 148.2 (*s*); 112.0 (*t*); 65.5 (*t*); 55.3 (*d*); 45.6 (*d*); 43.5 (*s*); 40.7 (*d*); 39.7 (*t*); 29.7 (*t*); 27.3 (*q*); 26.7 (*t*); 24.4 (*t*); 15.5 *(q*); 14.3 (*q*); 11.5 (*q*) ; minor 149.2 (*s*); 112.1 (*t*); 65.6 (*t*); 56.2 (*d*); 45.4 (*d*); 43.6 (*s*); 41.0 (*d*); 40.9 (*t*); 29.8 (*t*); 27.5 (*q*); 26.9 (*t*); 23.2 (*t*); 15.4 *(q);* 14.3 (*q*); 11.2 (*q*).
4) (-)-2-ethyl-4-((1*R'*,3*R'*)-3-ethyl-2,2-dimethylcyclopentyl)pent-4-en-1-ol
   a) *(1'R,3'R)-2, 2-dimethyl-3-ethylcyclopentylacetaldhehyde* Aldehyde (+)-3-ethyl-2,2-dimethyl-3-cyclopentene-1-acetaldehyde [described in C. Chapuis, R. Brauchli, Helv. Chim. Acta 1992, 75, 1527] (5.25g, 29.2 mmol) was hydrogenated under 5 bars of H₂ in AcOEt (25 ml) in the presence of 10% Pd/C (150 mg). The crude reaction mixture was filtered, concentrated and purified by CC/SiO₂ with cyclohexane/AcOEt 9:1 to afford the desired product in 57% yield.
      [α]_{D}²⁰= -17.5 (c = 3.8, CHCl₃)
      ¹H-NMR: 9.77 (*t, J* = 2.0, 1H); 2.48 (*dt, J* = 2.5, 15.9, 1H); 2.19 (*ddd, J* = 3.1, 10.1, 15.9, 1H); 1.90 *(m,* 2H); 1.46 *(m,* 1H); 1.32 (*m,* 1H); 1.20 (*m,* 3H); 1.02 (*m,* 1H); 0.91 (*s*, 3H); 0.90 *(t, J = 7,* 3H); 0.55 (*s*, 3H).
      ¹³C-NMR: 203.3 (*d*); 52.4 (*d*); 45.3 (*t*); 45.0 (*d*); 42.6 (*s*); 28.1 (*t*); 28.0 (*t*); 25.7 (*q*); 23.1 (*t*); 15.2 (*q*); 13.3 (*q*).
   b*) (-)-((1'R.3'R)-2-(2',2'-dimethyl-3'-ethyl-1'-cyclopentyl)-2-propenal*
      A mixture of (1'R,3'R)-2,2-dimethyl-3-ethylcyclopentylacetaldhehyde and aqueous formaldehyde (1.32 g, 15.85 mmol) was heated at reflux under N₂ and magnetic stirring. Dibutylamine (0.125g, 0.97 mmol) was added and after completion of the reaction, the cold mixture was extracted with Et₂O, washed with brine, then H₂O, dried (Na₂SO₄), concentrated and purified by column chromatography over SiO₂ with cyclohexane/AcOEt 97:3 to afford the desired product in 49% yield.
      [α]_{D}²⁰ = -7.5 (c = 3.7, CHCl₃)
      ¹H-NMR: 9.56 (*s*, 1H) ; 6.27 (*s*, 1H); 6.15 (*s*, 1H); 2.91 (*dd, J* = 9.7, 10.2, 1H) ; 1.94 (*m,* 1H) ; 1.71 (*m,* 2H) ; 1.48 (*m,* 2H) ; 1.28 *(m.* 1H) ; 1.04 (*m,* 1H) ; 0.90 (*t*, *J* = 7, 3H); 0.80 (*s*, 3H) ; 0.51 (*s*, 3H).
      ¹³C-NMR: 195.1 (*d*); 150.1 (*s*); 135.8 *(t);* 53.2 *(d);* 46.8 *(d);* 43.6 (*s*); 27.7 (*t*); 26.9 (*t*); 26.2 *(q);* 23.4 *(t);* 15.9 *(q);* 13.3 *(q).*
   *c) (-)-(1'R,3'R)2-(2',2'-dimethyl-3'-ethyl-1'-cyclopenthyl)-2-propen-1-ol* (-)-((1'R,3'R)-2-(2',2'-dimethyl-3'-ethyl-1'-cyclopentyl)-2-propenal (1.44g, 7.8 mmol) was reduced with LiAlH₄ (0.207 g, 5.46 mmol) in THF (20 ml) according to the procedure used under 1-b) in 97% yield.
      [α]_{D}²⁰ = -30.9, (c = 4.0, CHCl₃)
      ¹H-NMR: 5.24 (*d, J* = 1.5, 1H); 4.93 (*s*, 1H); 4.12 (*d, J* = 14.5, 1H); 4.04 (*d, J* = 14.5, 1H); 2.17 (*t, J*= 10.1, 1H); 1.88 (*m*, 1H); 1.72 (*dd, J* = 6.5, 8.2. 2H); 1.57 (brs, 1OH); 1.45 (*m*, 1H); 1.37 (*m,* 1H); 1.23 (*m,* 1H); 1.02 (*m,* 1H); 0.92 (*s*, 3H); 0.89 (*t*, *J* = 7, 3H); 0.56 (*s*, 3H).
      ¹³C-NMR: 149.0 (*s*); 110.8 (*t*); 66.8 (*t*); 54.1 (*d*); 53.1 (*d*); 43.5 (*s*); 27.5 (*t*); 26.9 (*t*); 26.7 (*q*); 23.3 (*t*); 16.0 (*q*); 13.3 (*q*).
   *d) methyl (1'R,3'R)-2-ethyl-4-(3'-ethyl-2',2'-dimethyl-1'-cyclopentyl)-4-pentenoate* Obtained in 72% yield as a 53:47 mixture of diastereoisomers from (-)-(1'R,3'R)2-(2',2'-dimethyl-3'-ethyl-1'-cyclopentyl)-2-propen-1-ol according to the procedure used under 1-c).
      [α]_{D}²⁰ = -22.8, (c = 3.8, CHCl₃)
      ¹H-NMR: major 4.91 (*s*, 1H); 4.77 (*s*, 1H); 3.63 (*s*, 3H); 2.55 (*m*, 1H); 2.37 (*m*, 1H); 2.15 (*m,* 2H); 1.87 (*m,* 1H); 1.66 (*m*, 2H); 1.55 (*m*, 2H); 1.45 (*m*, 1H); 1.36 (*m,* 1H); 1.20 (*m,* 1H); 1.00 (*m,* 1H); 0.93 (*s*, 3H); 0.90 (*t*, *J* = 7, 3H); 0.88 (*t*, *J* = 7, 3H); 0.53 (*s*, 3H);
      minor 4.91 (*s*, 1H); 4.81 (*s*, 1H); 3.67 (*s*, 3H); 2.50 (*m,* 1H); 2.37 (*m,* 1H); 2.15 (*m*, 2H); 1.87 (*m*, 1H); 1.66 (*m*, 2H); 1.55 (*m,* 2H); 1.45 (*m,* 1H); 1.36 (*m,* 1H); 1.20 (*m,* 1H); 1.00 (*m*, 1H); 0.92 (*s*, 3H); 0.88 (*t, J* = 7, 3H); 0.89 (t, *J* = 7, 3H); 0.54 (*s*, 3H).
      ¹³C-NMR: major 176.6 (*s*); 146.8 (*s*); 112.5 (*t*), 56.2 (*q*); 53.3 (*d*); 51.4 (*d*); 46.4 (*d*); 43.5 (*s*); 39.6 (*t*), 27.3 (*t*); 27.0 (*t*); 26.9 (*d*); 25.8 (*t*); 23.3 (*t*); 16.0 (*q*); 13.3 (*q*); 11.8 (*d*);
      minor 176.3 (*s*); 146.8 (*s*); 111.9 *(t);* 56.7 (*q*); 53.1 (*d*); 51.2 *(d);* 46.7 (*d*); 43.7 (*s*); 40.8 (*t*); 27.6 (*t*); 27.4 (*t*); 26.8 (*d*); 25.3 (*t*); 23.3 (*t*); 15.9 (*q*); 13.3 (*q*); 12.0 (*q*)*.*
   *e) (-)-2-ethyl-4-((1'R,3'R)-3-ethyl-2,2-dimnethylcyclopentyl)pent-4-en-1-ol* Methyl (1'R,3'R)-2-ethyl-4-(3'-ethyl-2',2'-dimethyl-1'-cyclopentyl)-4-pentenoate (1.2 g, 4.36 mmol) was reduced with LiAlH₄ (116 mg, 3.05 mmol) in Et₂O (12 ml) according to the procedure used under 1-d), to afford a 1.1:1 mixture of diastereoisomers in 72% yield.
      [α]_{D}²⁰ = -11.8, (c = 3.8, CHCl₃)
      ¹H-NMR: major 4.98 (*s*, 1H) ; 4.82 (*s*, 1H) ; 3.56 (*m*, 2H); 2.15 (*m*, 2H); 1.88 (*m*, 1H), 1.66 *(m,* 2H); 1.50 (brs, 1OH); 1.42 *(m,* 3H); 1.32 *(m,* 3H); 1.21 (*m*, 1H); 1.02 (*m*, 1H); 0.94 (s, 3H); 0.92 (*t*, *J* = 7, 3H); 0.89 *(s,* 3H); 0.89 (*t, J* = 7, 3H);
      minor 4.94 (*s*, 1H); 4.84 (*s*, 1H); 3.56 (*m*, 2H); 2.15 *(m,* 2H); 1.88 (*m*, 1H); 1.66 (*m*, 2H); 1.50 (brs, 1OH); 1.42 (*m*, 3H); 1.32 *(m,* 3H); 1.21 (*m*, 1H); 1.02 (*m*, 1H); 0.93 (*s*, 3H); 0.92 (*t*, *J* = 7, 3H); 0.91 (s, 3H); 0.89 (*t*, *J* = 7, 3H).
      ¹³C-NMR: major 149.0 (*s*); 112.1 (*t*); 65.6 (*t*); 55.4 (*d*); 53.3 (*d*); 43.5 (*s*); 40.7 (*d*); 39.8 (*t*); 27.5 (*t*); 27.2 (*t*); 27.0 (*q*); 24.4 (*t*); 23.3 (*t*); 16.0 (*d*); 13.3 (*q*); 11.5 (*q*);
      minor 148.0 (*s*); 112.1 (*t*); 65.4 (*t*); 56.3 (*d*); 53.2 (*d*); 43.6 (*s*); 41.1 (*d*); 40.9 (*t*); 27.5 (*t*); 27.4 (*t*); 26.9 (*q*); 23.3 (*t*); 23.2 (*t*); 16.0 (*q*); 13.3 (*q*); 11.2 (*q*).
5) (-)-2-ethyl-4-((1S,3R,5R)-1,2,2-trimethylbicyclo[3.1.0]hexan-3-yl)pent-4-en-1-ol
   *a) (*+*)*-*(1'S,3'S.5'R)-2-(1',2',2'-trimethylbicyclo[3.1.0]hex-3'-yl)-2-propenal* Bu₂NH (0.16g, 1.2 mmol) was added to a mixture of aldehyde (+)-(1*S*,3*R*,5*R*)-(1,2,2-trimethylbicyclo[3.1.0]hex-3-yl)acetaldehyde [see Helv. Chim. Acta 1998, 81, 1349] (4.3g, 26 mmol) and 36% H₂CO/H₂O (2.16g, 26 mmol) at reflux. After 3 hours the cold reaction mixture was extracted with Et₂O. The organic phase was washed to neutrality with H₂O, dried (Na₂SO₄), concentrated and bulb-to-bulb distilled to afford the desired product in 74% yield.
      Bp. 95°C/5.4 mbar α_{D}²⁰ = +12.1.
      ¹H-NMR: 9.55 (s, 1H); 6.2 (*s*, 1H); 6.15 (s, 1H); 2.64 (*dd, J* = 7.5, 12, 1H); 1.99 *(dt, J = 4,* 12, 1H); 1.60 (*dd*, *J* = 6.5, 12, 1H); 1.08 *(m,* 1H); 1.07 (*s*, 3H); 0.88 (*s*, 3H); 0.68 (*s*, 3H); 0.11 (*dd*, *J* = 4, 7.5, 1H).
      ¹³C-NMR: 195.0 (*s*); 149.7 (*s*); 136.9 (t); 41.9 (*s*); 40.8 (d); 31.0 (*s*); 30.4 (*t*); 23.3 (*q*); 22.2 (*d*); 21.4 (*q*); 17.5 (*q*); 13.7 (*t*).
   b) *(+)-(1'S,3'S,5'R)-2-(1',2',2'-trimethylbicyclo[3.1.0]hex-3'-yl]-2-propen-1-ol* A solution of aldehyde (+)-(1'S,3'S,5'R)-2-(1',2',2'-trimethylbicyclo[3.1.0]hex-3'-yl)-2-propenal (3.4g, 20 mmol) in Et₂O (10 ml) was added dropwise to a suspension of LiAlH₄ (0.72g, 20 mmol) in Et₂O (10 ml). After 3 hours H₂O (0.7 ml); 15% NaOH (0.7 ml); H₂O (5 ml), were added successively. The mixture was filtered, dried (Na₂SO₄), concentrated, and bulb-to-bulb distilled to afford pure the desired product in 73% yield.
      Bp: 93°C/3 mbar. α_{D}²⁰ = +0.3.
      ¹H-NMR: 5.20 (*s*, 1H); 4.85 (*s*, 1H); 4.04 (*AB*, *J* = 14, 36, 2H); 1.95 (*m*, 2H); 1.80 (brs, OH); 1.62 (*d*, *J* = 5.9, 1H); 1.05 (*s*, 3H); 1.03 (*m*, 1H); 0.98 (*s*, 3H); 0.74 (*s,* 3H); 0.52 (*t*, *J* = 4.2, 1H); 0.06 (*dd*, *J* = 4.2, 7.5, 1H).
      ¹³C-NMR: 148.4 (*s*); 111.0 (*t*); 66.5 (*t*); 47.2 (*d*); 41.8 (*s*); 31.1 (*s*); 30.0 (*t*); 23.8 (*q*); 22.1 (*d*); 20.9 (*q*); 17.4 (*q*); 13.7 (*t*).
   c) *(-)-methyl 2-ethyl-4-((1S,3R,5R)-1,2,2-trimethylbicyclo[3.1.0]hexan-3-yl)pent-4-enoate*
      Obtained in 52% yield according to the procedure used under 1-c), as a 1.2:1 mixture of diastereoisomers.
      Bp: 110°C/0.15 mbar. α_{D}²⁰ = -10.9 (c = 4.2, CHCl₃).
      ¹H-NMR: major 4.87 (*s*, 1H); 4.74 (*s*, 1H); 3.65 (*s*, 3H); 2.49 (*m*, 1H); 2.34 (*m*, 1H); 2.13 (*m*, 1H); 1.90 (*m,* 2H); 1.55 (*m*, 4H); 1.04 (*s*, 3H); 0.99 (*s*, 3H); 0.89 (*t*, *J* = 7, 3H); 0.73 (*s*, 3H); 0.51 (*m*, 1H); 0.04 (*m*, 1H);
      minor 4.86 (*s*, 1H); 4.70 (*s*, 1H); 3.63 (*s*, 3H); 2.49 (*m*, 1H); 2.34 (*m*, 1H); 2.13 (*m*, 1H); 1.90 (*m*, 2H); 1.55 (*m*, 4H); 1.04 (*s*, 3H); 1.02 (*s*, 3H); 0.91 (*t*, *J* = 7, 3H); 0.72 (*s*, 3H); 0.51 (*m*, 1H); 0.04 (*m*, 1H).
      ¹³C-NMR: major 176.5 (*s*); 146.2 (*s*); 112.7 (*t*); 51.3 (*q*); 49.5 (*d*); 46.0 (*d*); 42.0 (*s*); 39.2 (*t*); 31.0 (*s*); 30.2 (*t*); 25.4 (*t*); 23.8 (*q*); 22.1 (*d*); 20.9 (*q*); 17.4 (*q*); 13.8 (*t*)*;* 11.8 (*q*);
      minor 176.2 (*s*); 146.2 (*s*); 112.3 (*t*); 51.2 (*q*); 49.0 (*d*); 46.3 (*d*); 41.8 (*s*); 40.1 (*t*); 31.1 (*s*), 30.5 (*t*); 25.8 (*t*); 24.1 (*q*); 22.0 (*d*); 21.0 (*q*); 17.4 (*q*); 13.9 (*t*); 12.0 (*q*).
   d) *(-)-2-ethyl-4-((1S,3R,SR)-1,2,2-trirmthylbicyclo[3.1.0]hexan-3-yl)pent-4-en-1-ol* Obtained in 94% yield according to the procedure used for under 1-d), as a 52:48 mixture of diastereoisomers.
      Bp: 100°C/0.1 mbar. α_{D}²⁰ = -2.1 c = 4, CHCl₃.
      ¹H-NMR: major 4.94 (*s*, 1H); 4.77 (*s*, 1H); 3.54 (*m*, 2H); 2.10 (*m*, 1H); 1.97 (*m*, 2H); 1.90 (*m*, 1H); 1.61 (*m*, 2H); 1.45 (brs, OH); 1.42 (*s*, 3H); 1.30 (*m,* 2H); 1.04 (*s*, 3H); 1.03 (*m,* 1H); 0.92 (*t, J* = 7, 3H); 0.74 (*s*, 3H); 0.51 (*m,* 1H); 0.04 (*m*, 1H);
      minor 4.90 (*s*, 1H); 4.76 (*s*, 1H); 3.54 *(m,* 2H); 2.10 (*m*, 1H); 1.97 (*m*, 2H); 1.90 (*m*, 1H); 1.61 (*m,* 2H); 1.45 (brs, OH); 1.42 (*s*, 3H); 1.30 (*m*, 2H); 1.03 (*s*, 3H); 1.03 (*m*, 1H); 1.01 (*s*, 3H); 0.89 (t, *J* = 7, 3H); 0.51 (*m*, 1H); 0.04 (*m*, 1H).
      ¹³C-NMR: major 148.2 (*s*); 112.6 (*t*); 65.4 (*t*); 49.0 (*d*); 41.8 (*s*); 40.2 (*t*); 40.2 (*d*); 31.0 (*s*); 30.5 (*t*); 24.3 (*t*); 24.2 *(q);* 22.2 (*d*); 21.0 (*q*); 17.5 (*q*); 13.9 (*t*); 11.5 (*q*);
      minor 147.3 (*s*); 112.6 (*t*); 65.4 (*t*); 48.2 (*d*); 42.0 (*s*); 40.5 (*t*); 39.2 (*d*); 31.2 (*s*); 30.4 (*t*); 24.0 (*q*); 23.2 (*t*); 22.1 (*d*); 21.0 (*q*); 17.4 (*q*); 13.8 (*t*); 11.1 (*q*).

### Example 2

### Preparation of a perfuming composition

An "eau de toilette" for woman was prepared by admixing the following ingredients:

| Ingredient | Parts by weight |
|---|---|
| 10%* Aldehyde C 11 undecylenic | 10 |
| 10%* Cuminic aldehyde | 15 |
| Benzyl benzoate | 290 |
| Benzylacetone | 5 |
| Citronellol | 80 |
| 4-Cyclohexyl-2-methyl-2-butanol¹⁾ | 25 |
| (1'R,E)-2-Ethyl-4-(2',2',3'-trimethyl-3'-cyclo penten-1'-yl)-2-buten-1-ol¹⁾ | 10 |
| 10%* Damascone Beta | 20 |
| Phenylethyl formiate | 10 |
| Geraniol | 60 |
| Geranium essential oil | 30 |
| Clove essential oil | 10 |
| Gurjun baume | 480 |
| Ionone Beta | 5 |
| 10%* Isoeugenol | 20 |
| 10%* Methyl heptinecarbonate | 15 |
| 10%* Crystal moss | 15 |
| Muscenone delta²⁾ | 10 |
| 1%* Rose oxide | 10 |
| Patchouli oil | 20 |
| Phenethylol | 490 |
| 10%* Phenylethyl phenylacetate | 25 |
| 10%* 9-Decen-1-ol | 20 |
| Bulgarian rose essential oil | 10 |
| 10%* 2,3,3-Trimethyl-1-indanone | 10 |
| 10%* Methyl salicylate | 5 |
| | 1700 |

| | |
|---|---|
| * in dipropyleneglycol 1) origin : Firmenich SA, Geneva, Switzerland 2) 3-Methyl-(4)-cyclopentadecenone; origin : Firmenich SA, Geneva, Switzerland | |

The addition of 200 parts by weight of (1'R)-2-ethyl-4-(2',2',3'-trimethyl-3'-cyclopenten-1'-yl)-4-penten-1-ol to the above-described eau de toilette conferred a clear, rising connotation of the head note of sandalwood, woody-sandalwood, type, not at all sandalwood milk or sandal Mysore. This connotation amplified and completed the sandal note provided by the (1'R.E)-2-ethyl-4-(2',2',3'-trimethyl-3'-cyclopenten-1'-yl)-2-buten-1-ol present in the original eau de toilette. Furthermore, the invention's compound married very well with the rose connotation of the original eau de toilette, transforming the original floral note into a very nice and elegant woody-rose note.

When, instead of the invention's compound it was added the same amount of the prior art 2-methyl-4-(2',2'-dimethyl-3'-methyl-3'-cyclopenten-1'-yl)-4-penten-1-ol, the fragrance obtained was much more dry and cedar wood like, as well as less complex and the original floral note of the eau de toilette was not modified, rendering the floral aspect of the new fragrance much less appealing.

### Example 3

### Preparation of a perfuming composition

An "eau de toilette" for man was prepared by admixing the following ingredients:

| Ingredient | Parts by weight |
|---|---|
| Isoeugenyl acetate | 5 |
| Dimethyl benzyl carbinyl acetate | 20 |
| Geranyl acetate | 50 |
| Cinnamic alcohol | 300 |
| Hexylcinnamic aldehyde | 300 |
| 10%* Chinese anis essential oil | 20 |
| 50%* Styrax essential oil | 600 |
| 10%* Methyl benzoate | 15 |
| Cinnamon essential oil | 30 |
| Cardamom essential oil | 130 |
| 10%* Cashmeran^{® 1)} | 75 |
| 10%* Cetalox^{®2)} | 30 |
| 10%* Cis-3-Hexenol | 10 |
| Citronellol | 80 |
| Copahu essential oil | 60 |
| Coumarine | 20 |
| 10%* Cumin | 30 |
| Cypress essential oil | 80 |
| 3-(3-Isopropyl-1-phenyl)butanal | 20 |
| Gaiac essential oil | 20 |
| Geraniol | 10 |
| Clove essential oil | 440 |
| Hedione^{®3)} | 100 |
| Heliotropine ⁴⁾ | 40 |
| 10%* Indol | 10 |
| Ionone Beta | 35 |
| 10%* Labdanum essential oil | 150 |
| Lavender essential oil | 250 |
| Nutmeg essential oil | 100 |
| Nirvanol^{®5)} | 100 |
| Phenethylol | 180 |
| Phenylethyl phenylacetate | 55 |
| 10%* Ethyl phenylacetate | 10 |
| 10%* 9-Decen-1-ol | 75 |
| Bulgarian essential oil | 20 |
| Vanilline | 5 |
| Vertofix^{®} Coeur IFF⁶⁾ | 325 |
| | 3800 |

| | |
|---|---|
| * in dipropyleneglycol 1) 1,2,3,5,6,7-Hexahydro-1,1,2,3,3-pentamethyl-4-indenone; origin : IFF, USA 2) (-)-8,12-Epoxy-13,14,15,16-tetranorlabdane; origin : Firmenich SA, Geneva, Switzerland 3) Methyl dihydrojasmonate; origin: Firmenich SA, Geneva, Switzerland 4) 1,3-Benzodioxole-5-carbaldehyde; origin: Firmenich SA, Geneva, Switzerland 5) 3,3-Dimethyl-5-(2,2,3-trimethyl-3-cyclopenten-1-yl)-4-penten-2-ol; origin: Firmenich SA, Geneva, Switzerland 6) Methyl cedryl ketone; origin : IFF, USA | |

The addition of 100 parts by weight of (1'R)-2-ethyl-4-(2',2',3'-trimethyl-3'-cyclopenten-1'-yl)-4-penten-1-ol to the above-described eau de toilette clearly boosted the head note of sandalwood aspect and completed the sandal note provided by the Nirvanol^{®} present in the original eau de toilette. Furthermore, the invention's compound married very well with, and boosted, the floral-rose connotation of the original eau de toilette.

When, instead of the invention's compound it was added the same amount of the prior art 2-methyl-4-(2',2'-dimethyl-3'-methyl-3'-cyclopenten-1'-yl)-4-penten-1-ol, the fragrance obtained was much more dry and cedar wood like, and the overall fragrance was no more floral than the original eau de toilette.

## Claims

1. A compound of formula 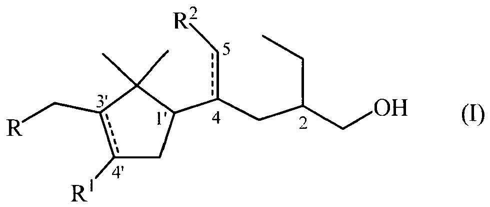 wherein R is a hydrogen atom or a methyl group;
R² represents a hydrogen atom and the bond between the carbon atoms 4 and 5 is a double bond, or R² represents a CH₂ group forming with the carbon atoms 4 and 5 a cyclopropane ring; and
R¹ represents a hydrogen atom and the bond between the carbon atoms 3' and 4' is a single or double bond, or R¹ represents a CH₂ group forming with the carbon atoms 3' and 4' a cyclopropane ring;
in the form of any one of its enantiomers or in the form of a mixture of said enantiomers.

2. A compound according to claim 1, **characterized in that** said compound is of formula 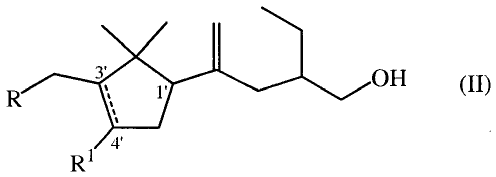 wherein R is a hydrogen atom or a methyl group; and
R¹ represents a hydrogen atom and the bond between the carbon atoms 3' and 4' is a single or double bond, or R¹ represents a CH₂ group forming with the carbon atoms 3' and 4' a cyclopropane ring.

3. A compound according to claim 1, **characterized in that** said compound has the carbon atom 1' with a stereo configuration of the type (R), and an enantiomeric excess of above 50 %*.*

4. A compound according to claim 1, **characterized in that** said compound is (1'R)-2-ethyl-4-(2',2',3'-trimethyl-3'-cyclopenten-1'-yl)-4-penten-1-ol, 2-ethyl-4-(2',2',3'-trimethyl-3'-cyclopenten-1'-yl)-4-penten-1-ol, (1'R)-2-ethyl-4-(3'-ethyl-2',2'-dimethyl-3'-cyclopenten-1'-yl)-4-penten-1-ol or (1'R)-2-ethyl-4-(2',2',3'-trimethyl-1'-cyclopentyl)-4-penten-1-ol.

5. Use as perfuming ingredient of a compound of formula (I), as defined in any one of claims 1 to 4.

6. A perfuming composition comprising
i) at least a compound as defined in any one of claims 1 to 4;
ii) at least one ingredient selected from the group consisting of a perfumery carrier and a perfumery base; and
iii) optionally at least one perfumery adjuvant.

7. A perfuming consumer product comprises:
i) at least one compound of formula (I), as defined in any one of claims 1 to 4; and
ii) a fine or functional perfumery base.

8. A perfumed article according to claim 7, **characterized in that** the fine or functional perfumery base is a perfume, a fabric care product, a body-care product, an air care product or a home care product.

9. A perfumed article according to claim 7, **characterized in that** the fine or functional perfumery base is a fine perfume, a cologne, an after-shave lotion, a liquid or solid detergent, a fabric softener, a fabric refresher, an ironing water, a paper, a bleach, a shampoo, a coloring preparation, a hair spray, a vanishing cream, a deodorant or antiperspirant, a perfumed soap, shower or bath mousse, oils or gel, a hygiene product, an air freshener, a "ready to use" powdered air freshener, a wipe, a dish detergent or hard-surface detergent.

10. A compound of formula 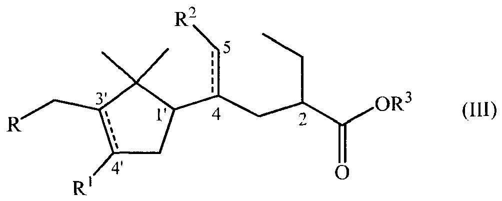 wherein R is a hydrogen atom or a methyl group;
R² represents a hydrogen atom and the bond between the carbon atoms 4 and 5 is a double bond, or R² represents a CH₂ group forming with the carbon atoms 4 and 5 a cyclopropane ring;
R¹ represents a hydrogen atom and the bond between the carbon atoms 3' and 4' is a single or double bond, or R¹ represents a CH₂ group forming with the carbon atoms 3' and 4' a cyclopropane ring; and
R³ represents a C₁₋₄ alkyl group;
in the form of any one of its enantiomers or in the form of a mixture of said enantiomers.

## Patentansprüche

1. Verbindung der Formel 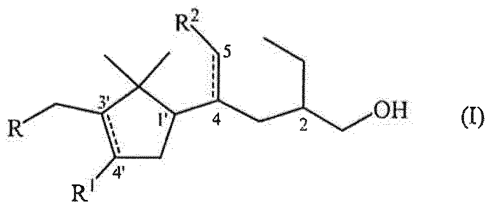 wobei R ein Wasserstoffatom oder eine Methylgruppe ist;
R² ein Wasserstoffatom darstellt und die Bindung zwischen den Kohlenstoffatomen 4 und 5 eine Doppelbindung ist, oder R² eine CH₂-Gruppe darstellt, die mit den Kohlenstoffatomen 4 und 5 einen Cyclopropanring bildet; und
R¹ ein Wasserstoffatom darstellt und die Bindung zwischen den Kohlenstoffatomen 3' und 4' eine Einfach- oder Doppelbindung ist, oder R¹ eine CH₂-Gruppe darstellt, die mit den Kohlenstoffatomen 3' und 4' einen Cyclopropanring bildet;
in der Form von einem ihrer Enantiomere oder in der Form von einem Gemisch der Enantiomere.

2. Verbindung gemäß Anspruch 1, **dadurch gekennzeichnet, dass** die Verbindung die Formel 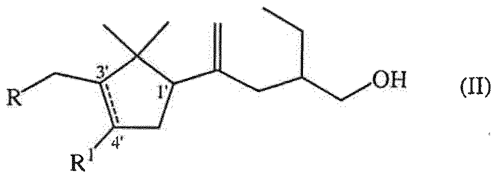 hat, wobei R ein Wasserstoffatom oder eine Methylgruppe ist; und
R¹ ein Wasserstoffatom darstellt und die Bindung zwischen den Kohlenstoffatomen 3' und 4' eine Einfach- oder Doppelbindung ist, oder R¹ eine CH₂-Gruppe darstellt, die mit den Kohlenstoffatomen 3' und 4' einen Cyclopropanring bildet.

3. Verbindung gemäß Anspruch 1, **dadurch gekennzeichnet, dass** die Verbindung das Kohlenstoffatom 1' mit einer Stereokonfiguration des Typs (R) und einen enantiomeren Überschuss von über 50% aufweist.

4. Verbindung gemäß Anspruch 1, **dadurch gekennzeichnet, dass** die Verbindung (1'R)-2-Ethyl-4-(2',2',3'-trimethyl-3'-cyclopenten-1'-yl)-4-penten-1-ol, 2-Ethyl-4-(2',2',3'-trimethyl-3'-cyclopenten-1'-yl)-4-penten-1-ol, (1'R)-2-Ethyl-4-(3'-ethyl-2',2'-dimethyl-3'-cyclopenten-1'-yl)-4-penten-1-ol oder (1'R)-2-Ethyl-4-(2',2',3'-trimethyl-1'-cyclopentyl)-4-penten-1-ol ist.

5. Verwendung als parfümierender Bestandteil einer Verbindung der Formel (I), wie in einem der Ansprüche 1 bis 4 definiert.

6. Parfümierende Zusammensetzung, umfassend
i) mindestens eine Verbindung, wie in einem der Ansprüche 1 bis 4 definiert;
ii) mindestens einen Bestandteil, ausgewählt aus der Gruppe, bestehend aus einem Träger für Parfümfabrikation und einer Basis für Parfümfabrikation; und
iii) gegebenenfalls mindestens einen Hilfsstoff für Parfümfabrikation.

7. Parfümierendes Verbraucherprodukt, das umfasst:
i) mindestens eine Verbindung der Formel (I), wie in einem der Ansprüche 1 bis 4 definiert; und
ii) eine Basis für feine oder funktionelle Parfümfabrikation.

8. Parfümierter Gegenstand gemäß Anspruch 7, **dadurch gekennzeichnet, dass** die Basis für feine oder funktionelle Parfümfabrikation ein Parfüm, ein Gewebepflegeprodukt, ein Körperpflegeprodukt, ein Luftpflegeprodukt oder ein Hauspflegeprodukt ist.

9. Parfümierter Gegenstand gemäß Anspruch 7, **dadurch gekennzeichnet, dass** die Basis für die Fabrikation von feinen oder funktionellen Parfüms ein feines Parfüm, ein Kölnischwasser, eine Aftershavelotion, ein flüssiges oder festes Detergens, ein Weichspüler, ein Gewebeauffrischer, ein Bügelwasser, ein Papier, ein Bleichmittel, ein Shampoo, eine Farbzubereitung, ein Haarspray, eine Tagescreme, ein Deodorant oder Antiperspirant, eine parfümierte Seife, Dusch- oder Badeschaum, -öle oder -gel, ein Hygieneprodukt, ein Luftverbesserer, ein "gebrauchsfertiger" pulverförmiger Luftverbesserer, ein Wischtuch, ein Geschirrspülmittel oder Detergens für harte Oberflächen ist.

10. Verbindung der Formel 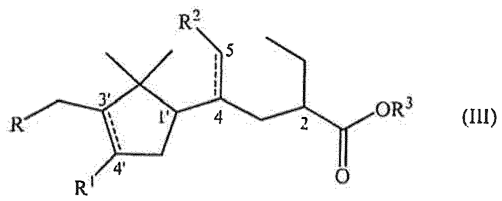 wobei R ein Wasserstoffatom oder eine Methylgruppe ist;
R² ein Wasserstoffatom darstellt und die Bindung zwischen den Kohlenstoffatomen 4 und 5 eine Doppelbindung ist, oder R² eine CH₂-Gruppe darstellt, die mit den Kohlenstoffatomen 4 und 5 einen Cyclopropanring bildet;
R¹ ein Wasserstoffatom darstellt und die Bindung zwischen den Kohlenstoffatomen 3' und 4' eine Einfach- oder Doppelbindung ist, oder R¹ eine CH₂-Gruppe darstellt, die mit den Kohlenstoffatomen 3' und 4' einen Cyclopropanring bildet; und
R³ eine C₁₋₄-Alkylgruppe darstellt;
in der Form von einem ihrer Enantiomere oder in der Form von einem Gemisch der Enantiomere.

## Revendications

1. Composé de formule 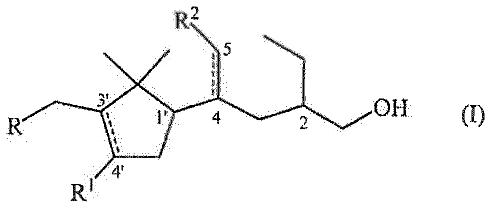 dans laquelle R est un atome d'hydrogène ou un groupe méthyle;
R² représente un atome d'hydrogène et la liaison entre les atomes de carbone 4 et 5 est une double liaison, ou R² représente un groupe CH₂ formant avec les atomes de carbone 4 et 5 un cycle cyclopropane; et
R¹ représente un atome d'hydrogène et la liaison entre les atomes de carbone 3' et 4' est une liaison simple ou une double liaison, ou R¹ représente un groupe CH₂ formant avec les atomes de carbone 3' et 4' un cycle cyclopropane;
sous la forme de l'un quelconque de ses énantiomères ou sous la forme d'un mélange desdits énantiomères.

2. Composé selon la revendication 1, **caractérisé en ce que** ledit composé a pour formule 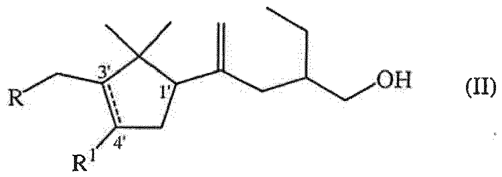 dans laquelle R est un atome d'hydrogène ou un groupe méthyle; et
R¹ représente un atome d'hydrogène et la liaison entre les atomes de carbone 3' et 4' est une liaison simple ou une double liaison, ou R¹ représente un groupe CH₂ formant avec les atomes de carbone 3' et 4' un cycle cyclopropane.

3. Composé selon la revendication 1, **caractérisé en ce que** ledit composé a l'atome de carbone 1' dont la configuration stéréochimique est du type (R), et l'excès énantiomérique supérieur à 50%.

4. Composé selon la revendication 1, **caractérisé en ce que** ledit composé est le (1'R)-2-éthyl-4-(2',2',3'-triméthyl-3'-cyclopentén-1'-yl)-4-pentén-1-ol, le 2-éthyl-4-(2',2',3'-triméthyl-3'-cyclopentén-1'-yl)-4-pentén-1-ol, le (1'R)-2-éthyl-4-(3'-éthyl-2',2'-diméthyl-3'-cyclopentén-1'-yl)-4-pentén-1-ol ou le (1'R)-2-éthyl-4-(2',2',3'-triméthyl-1'-cyclopentyl)-4-pentén-1-ol.

5. Utilisation, en tant qu'ingrédient parfumant, d'un composé de formule (I), tel que défini dans l'une quelconque des revendications 1 à 4.

6. Composition parfumante comprenant
i) au moins un composé tel que défini dans l'une quelconque des revendications 1 à 4;
ii) au moins un ingrédient choisi dans le groupe constitué d'un véhicule de parfumerie et d'une base de parfumerie; et
iii) éventuellement, au moins un adjuvant d'usage courant en parfumerie.

7. Produit de consommation parfumant qui comprend:
i) au moins un composé de formule (I), tel que défini dans l'une quelconque des revendications 1 à 4; et
ii) une base de parfumerie fine ou fonctionnelle.

8. Article parfumé selon la revendication 7, **caractérisé en ce que** la base de parfumerie fine ou fonctionnelle est un parfum, un produit d'entretien pour textile, un produit de soin corporel, un produit de traitement de l'air, ou un produit d'entretien ménager.

9. Article parfumé selon la revendication 7, **caractérisé en ce que** la base de parfumerie fine ou fonctionnelle est un parfum fin, une eau de Cologne, une lotion après-rasage, un détergent solide ou liquide, un adoucissant pour textile, une eau de linge, une eau de repassage, un papier, un agent de blanchiment, un shampooing, une préparation colorante, une laque capillaire, une crème de jour, un déodorant ou un anti-transpirant, un savon parfumé, une mousse, une huile ou un gel de bain ou de douche, un produit d'hygiène, désodorisant d'air ambiant, un désodorisant d'air ambiant en poudre « prêt à l'emploi », une lingette, un détergent pour la vaisselle ou un détergent pour surfaces dures.

10. Composé de formule 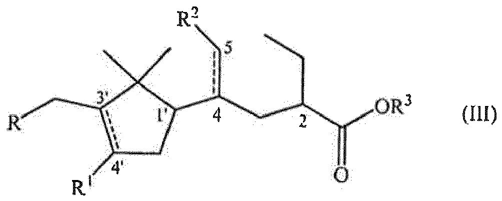 dans laquelle R est un atome d'hydrogène ou un groupe méthyle;
R² représente un atome d'hydrogène et la liaison entre les atomes de carbone 4 et 5 est une double liaison, ou R² représente un groupe CH₂ formant avec les atomes de carbone 4 et 5 un cycle cyclopropane;
R¹ représente un atome d'hydrogène et la liaison entre les atomes de carbone 3' et 4' est une liaison simple ou une double liaison, ou R¹ représente un groupe CH₂ formant avec les atomes de carbone 3' et 4' un cycle cyclopropane; et
R³ représente groupe alkyle en C₁₋₄;
sous la forme de l'un quelconque de ses énantiomères ou sous la forme d'un mélange desdits énantiomères.
